# EUROPÄISCHE PATENTANMELDUNG

(11) **EP 1 321 108 A1**
(43) Veröffentlichungstag der Anmeldung: **25.06.2003**
(21) Anmeldenummer: 02028411.3
(22) Anmeldetag: 18.12.2002
(51) Int. Cl.: A61C 1/18, A61B 1/247

(54) **Zahnärztliches Arbeitsgerät**

(30) Priorität: 20.12.2001 AT 9632001 U
(71) Anmelder: Rosenstatter, Otto, 5164 Seeham (AT)
(72) Erfinder: Rosenstatter, Otto, 5164 Seeham (AT)
(74) Vertreter: Hofinger, Stephan

(57) **Zusammenfassung**

Zahnärztliches Arbeitsgerät, mit einem ein angetriebenes Werkzeug 5) aufnehmenden Handstück (15), welches durch einen Leitungen enthaltenden Schlauch (14) mit einem Versorgungsgerät (28) drehbar verbunden ist.

## Beschreibung

Die Erfindung bezieht sich auf ein zahnärztliches Arbeitsgerät, mit einem ein angetriebenes Werkzeug aufnehmenden Handstück, welches durch einen Leitungen enthaltenden Schlauch mit einem Versorgungsgerät verbunden ist.

Zahnärztliche Handstücke werden üblicherweise von Luft und Wasser zur Bildung eines Sprays durchflossen. Außerdem gibt es Vorschläge, entlang des Handstückes einen Bildleiter zu führen, um das Operationsfeld aufnehmen zu können. Damit diese Maßnahme sinnvoll ist, muß eine Ausleuchtung des interessierenden Bereiches erfolgen, welche durch mit dem Handstück verbundene Lichtleiter bewirkt wird. Der mit dem Handstück verbundene Motor benötigt eine Energiezufuhr in Form von Druckluft oder elektrischer Energie.

Damit der Zahnarzt das Handstück um seine Längsachse drehen kann, sind üblicherweise zwischen Handstück und Motorgehäuse oder zwischen dem Motorgehäuse und einem am Ende des Schlauches fest verbundenen Adapterdrehkupplungen für die verschiedenen Leitungen vorgesehen. Mit steigender Zahl an Medien- und Signalleitungen wird es immer schwieriger, eine gut funktionierende Drehkupplung der beschriebenen Art zu konstruieren. Die Erfindung macht Drehkupplungen im Bereich des Handstückes überflüssig, indem vorgesehen wird, daß der Schlauch drehbar mit dem Versorgungsgerät verbunden ist. Dort ist ausreichend Platz für die Ausbildung der Drehkupplung.

Damit der Zahnarzt beim Verdrehen des Handstückes durch den Verdrehwiderstand des Schlauches nicht behindert wird, ist vorzugsweise vorgesehen, daß im Versorgungsgerät ein Antrieb zur Drehung des Schlauches vorgesehen ist. Der Zahnarzt braucht also nur so wie die für die Lenkung von Kraftfahrzeugen üblich ist, die gewünschte Drehbewegung einleiten und der im Versorgungsgerät vorgesehene Antrieb liefert das zur Verdrehung des Schlauches notwendige Drehmoment. Für die konstruktive Durchführung dieses Gedankens gibt es hinreichend Vorbilder im Stand der Technik. Im konkreten Fall ist vorteilhafterweise vorgesehen, daß zur Steuerung des Antriebs zwei Schalter vorgesehen sind, welche durch einen Schwenkteil betätigbar sind, der in einem mit dem Schlauch drehfest verbundenen Gehäuse gelagert ist.

Da aufgrund des drehbaren Schlauches und der ins Versorgungsgerät verlegten Kupplungen eine ungewöhnlich hohe Anzahl von Leitungen bis zur Werkzeugaufnahme geführt werden können, sind in einer bevorzugten Ausführung im Handstück auch Leitungen zum Ablesen von am Werkzeug angebrachten Informationen geführt. Diese sind insbesondere durch zwei Informationsspeicherringe gebildet, die Informationen zur Drehzahl sowie zum Drehmoment des Werkzeugs beinhalten.

Weitere Einzelheiten der Erfindung werden anschließend anhand der Zeichnung erläutert. In dieser sind
- Fig. 1: die Gesamtdarstellung eines Ausführungsbeispieles,
- Fig. 2: eine Vergrößerung des rechten Teiles in Fig. 1, teilweise im Schnitt,
- Fig. 3: eine Darstellung der Teile der linken Seite von Fig. 1,
- Fig. 4: ein Blick auf die Schnittstelle zwischen Handstück und Motorgehäuse in Richtung des Handstückes,
- Fig. 5: eine Darstellung entsprechend Fig. 5 in Richtung des Motorgehäuses,
- Fig. 6 und 7: zwei Darstellung eines Werkzeuges, und
- Fig. 8 bis 10: Längsschnitte durch drei verschiedene Ausführungen des in Fig. 3 gezeigten Leitungsträgers.

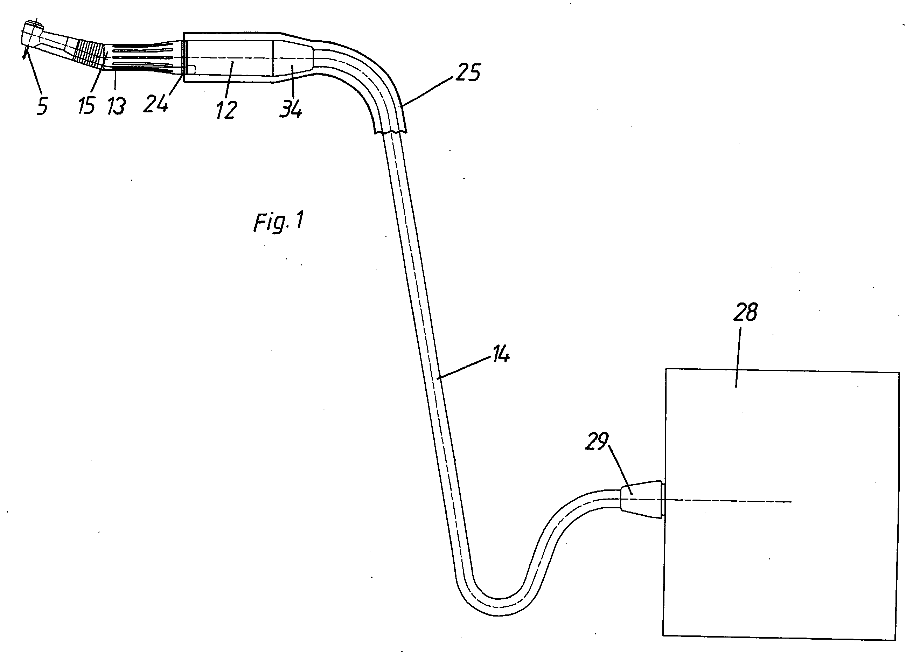

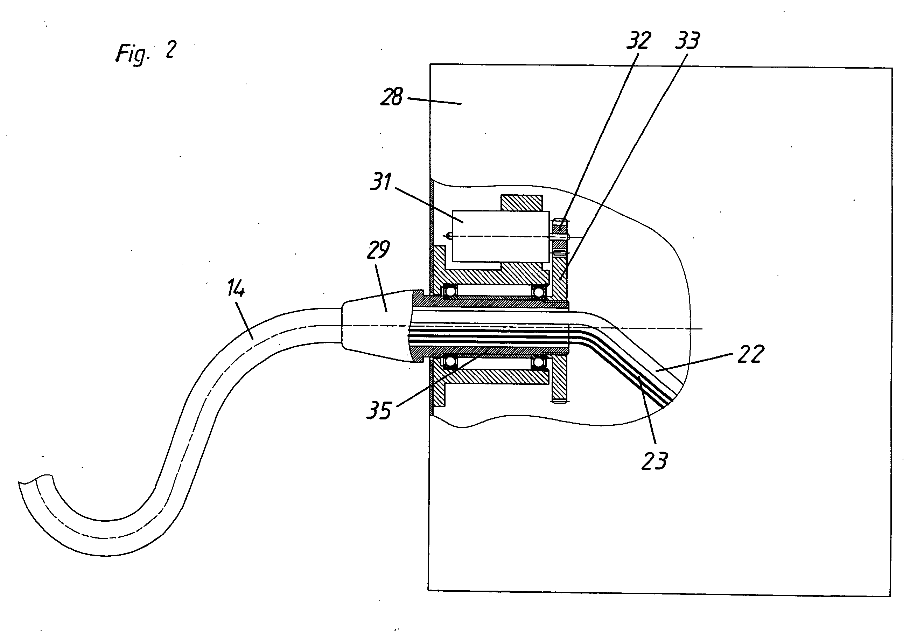

Das in Fig. 1 dargestellte zahnärztliche Arbeitsgerät umfaßt als wesentliche Teile ein Gehäuse 12 zur Aufnahme eines nicht dargestellten Motors und ein Handstück 15 zur Aufnahme eines Behandlungswerkzeuges 5, beispielsweise eines Bohrers. Zur Versorgung des Motors mit Energie und zur Rückführung von Signalen von der Präparationsstelle sind in einem verwindungssteifen Schlauch 14 Leitungen, insbesondere für Luft und Wasser, vorgesehen. Der Schlauch 14 ist mit dem Gehäuse 12 über ein Kupplungsstück 34 drehfest verbunden. Wie Fig. 2 zeigt, verbindet eine Steckkupplung 29 den Schlauch 14 mit dem Versorgungsgerät 28. Erfindungswesentlich ist es, daß diese Verbindung drehbar ist, was dadurch erreicht wird, daß die mit dem Schlauch 14 verbundene Hülse 35, welche unter anderem die Leitungen 22, 23 für Luft und Wasser umfaßt, drehbar gelagert ist.

Das Gehäuse 12 für den Motor und das Handstück 15 sind voneinander trennbar, was von erheblicher praktischer Bedeutung ist: Klemmt man in die Schnittstelle 24 zwischen diesen beiden Elementen einen dünnen Plastikschlauch 25, so genügt es, nach jeder Behandlung, das Handstück 15 und das Behandlungswerkzeug 5 zu autoklavieren. Das Gehäuse 12 und der Schlauch 14 hingegen sind durch den Plastikschlauch 25 geschützt, welcher nach der Behandlung weggeworfen und durch einen neuen Plastikschlauch ersetzt wird.

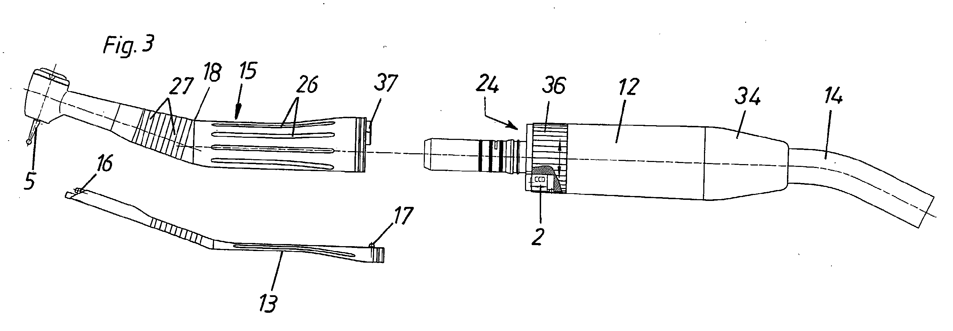

Fig. 3 zeigt, wie das Handstück 15 entlang der Schnittstelle 24 vom Gehäuse 12 trennbar ist, welches den Antrieb für das Werkzeug 5 und eine CCD-Kamera 2 enthält. Am Gehäuse 12 ist ein verdrehbarer Regelring 36 angeordnet, dessen Verdrehung die CCD-Kamera 2 axial verschiebt und somit den Fokus verstellt. Vom Handstück 15 ist ein Leitungsträger 13 abnehmbar, welcher im Betriebszustand mittels der Nase 16 und des Magneten 17 mit dem Handstück 15 verbunden ist. Dieser weist Längsrillen 26 auf, damit der Arzt das Instrument drehsicher halten kann. Die Querrillen 27 dienen der axialen Abstützung der Hand. Die Rillen 26 und 27 sind so ausgebildet, daß sie durch Bewegung in einer Richtung von Grobschmutz befreit werden können. Die Abnehmbarkeit des Leitungsträgers 13 ermöglicht es, ihn anders als durch Autoklavieren, beispielsweise durch chemische Desinfektionsmittel steril zu machen.

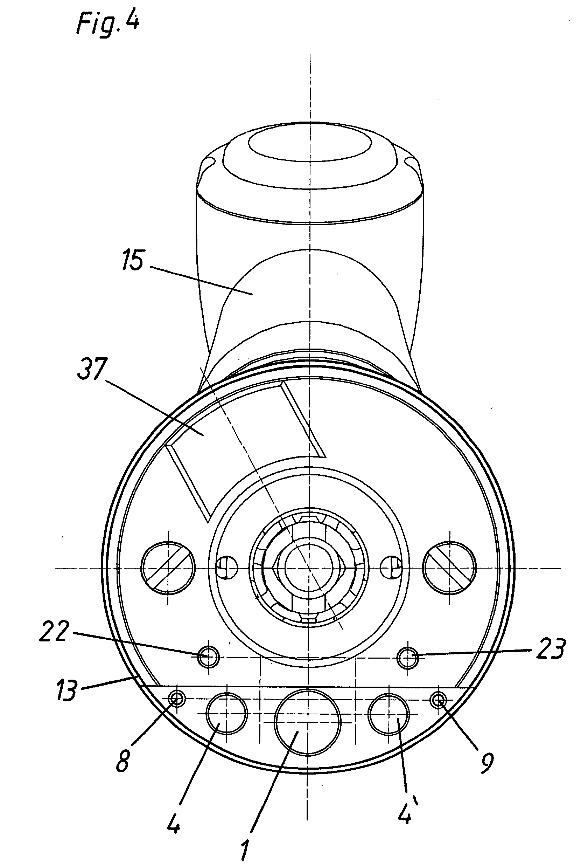

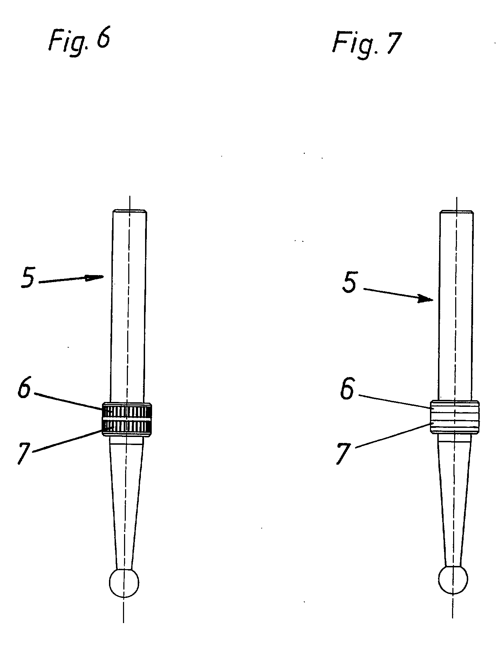

Aus Fig. 4 geht hervor, daß die Leitung 22 für die Luft und die Leitung 23 für das Wasser zur Erzeugung eines kühlenden Sprühnebels im Handstück 15 geführt sind. Im abnehmbaren Leitungsträger 13 befindet sich ein Bildleiter 1, welcher von der Behandlungsstelle zur CCD-Kamera 2 führt, sowie Lichtleiter 4 und 4', welche diese Behandlungsstelle beleuchten. Weiters sind Leitungen 8 und 9 im Leitungsträger 13 angeordnet, deren Funktion später anhand von Fig. 6 und 7 erläutert wird.

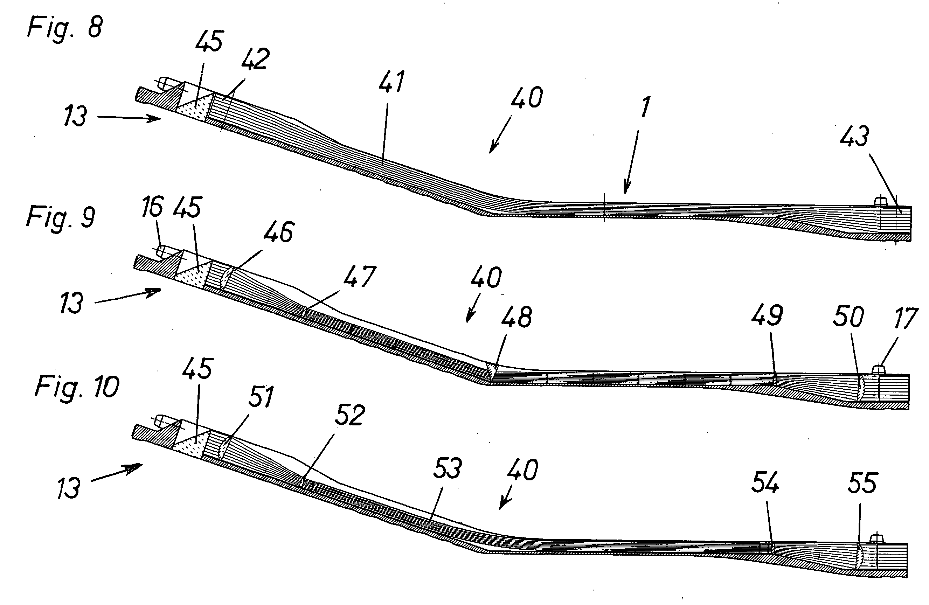

Die Fig. 8 bis 10 zeigen Längsschnitte durch den Bildleiter 1 in drei verschiedenen Ausführungsformen des abnehmbaren Leitungsträgers 13. Nach Fig. 8 ist der Bildleiter 1 durch einen Glasleiter 41 gebildet, der an einem Prisma 45 am Eingang 42 sowie am Ausgang 43 zur CCD-Kamera 2 einen kreisrunden Querschnitt, und in dem den Knick 18 des Handstückes 15 übergreifenden Zwischenbereich 40 hingegen einen ovalen Querschnitt aufweist.

Fig. 9 zeigt eine Ausführung, in der der vom Eingangsprisma 45 um etwa 90° umgelenkte Bildstrahl durch die nachgeordneten Linsen 46 und 47 im Durchmesser reduziert wird.

Der reduzierte Bildstrahl wird von einem dem Knick 18 des Handstücks 15 zugeordneten Umlenkprisma 48 auf ein weiteres Linsenpaar 49 und 50 gerichtet, das den Bildstrahl wieder öffnet und zur CCD-Kamera 2 weiter führt.

Fig. 10 zeigt eine Kombination der beiden Ausführungen von Fig. 8 und 9. Auch hier wird der Bildstrahl im Eingangsprisma 45 um etwa 90° umgelenkt und durch ein Lisenpaar 51 und 52 im Durchmesser reduziert. An die Linse 52 schließt ein dünner Glasleiter 53 an, der den Bildstrahl über den den Knick 18 des Handstücks 15 übergreifenden Zwischenbereich 40 zu dem zweiten Linsenpaar 54, 55 führt. Dieses öffnet den Bildstrahl wieder auf die für die CCD-Kamera 2 erforderliche Größe.

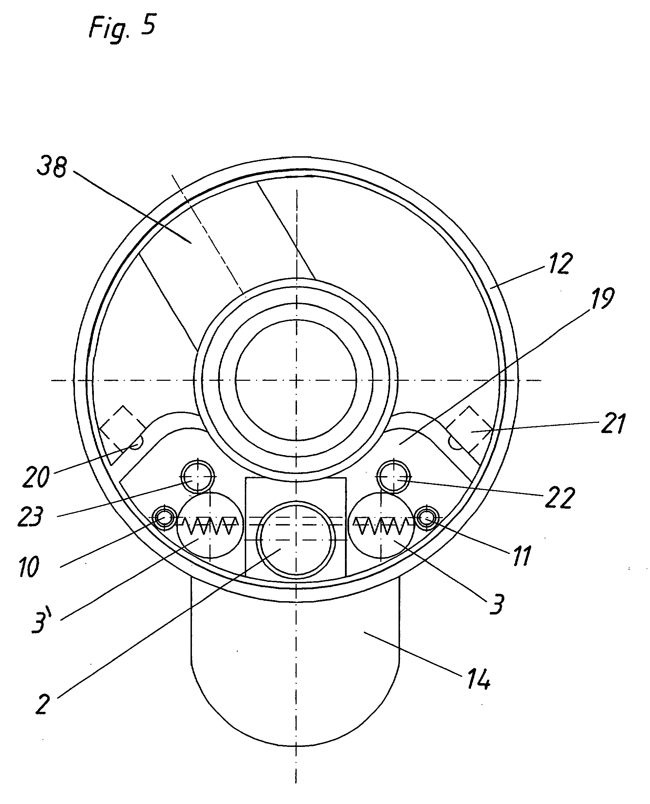

Die durch das Handstück geführten Leitungen 22 und 23 setzen sich mittels Steckverbindungen im Schwenkteil 19 fort, welcher im Gehäuse 12, wie aus Fig. 5 ersichtlich, gelagert ist. Die Lichtleiter 4, 4' des Handstücks 15 enden vor im Schwenkteil 19 angeordneten Glühlampen 3, 3'. Der Bildleiter 1 mündet in die CCD-Kamera 2, welche ihrerseits die umgewandelten Signale über den Schlauch 14 zum Versorgungsgerät 28 leitet. Die Bilddaten können auch über Funk berührungslos auf einen Bildschirm od. dgl. übertragen werden.

Verdreht der Zahnarzt geringfügig das Handstück 15, so folgt der Schwenkteil 19 dieser Bewegung. Das zur Verdrehung des Gehäuses 12 und des Schlauches 14 notwendige Moment wird jedoch nicht durch die Hand des Zahnarztes erzeugt, sondern durch den aus Fig. 2 ersichtlichen Motor 31. Dies wird dadurch erreicht, daß sich eine geringfügige Verdrehung des Schwenkteiles 19 von beispielsweise 5° auf den Schalter 20 oder 21 überträgt und über eine Signalleitung eine Drehung des Motors 31 in der geeigneten Drehrichtung auslöst. Dieser dreht mittels der Zahnräder 32 und 33 die Hülse 35, und damit den Schlauch 14, sodaß der Zahnarzt die Drehung nur veranlassen, aber nicht durchführen muß. Der Schlauch 14 folgt also der Bewegung des Zahnarztes synchron durch den ganzen Winkelbereich von 360°.

Der besondere Vorteil der erfindungsgemäßen Ausführung liegt darin, daß der kritische Übergang der Leitungen vom drehbaren auf den feststehenden Teil aus dem zu kleinen und filigranen Bereich des Handstücks 15 bzw. des Gehäuses 12 nach hinten an das Ende des Verbindungsschlauches gelegt wird, also an eine Stelle, an der im Normalfall wesentlich mehr Raum für die Lagerung und Abdichtung der einzelnen Elemente gegeben ist. Daher kann die Zahl der durch das Arbeitsgerät bzw. das Handstück geführten Leitungen ungewöhnlich hoch gemacht werden.

Insbesondere ist es dadurch auch möglich, am Werkzeug 5, wie in Fig. 6 und 7 dargestellt, je einen Informationsspeicherring für die maximal zulässige Drehzahl 6 und für das maximal zulässige Drehmoment 7 vorzusehen und die dort gespeicherten Informationen über zusätzliche Leitungen 8, 9 durch das Handstück einer Auswerteeinrichtung zuzuführen. Die Informationen über Drehzahl und Drehmoment können beispielsweise, wie in Fig. 6 gezeigt, jeweils als Barcode auf den Ringen 6, 7 vorgesehen sein; es sind aber auch andere Möglichkeiten denkbar, beispielsweise, daß die jeweiligen Informationen in einem Magneten (Fig. 7), einem Transponder, einer Speicherfolie oder einem elektronischen Papier gespeichert sind.

Für das Lesen der beiden Barcodes für die Drehzahl 6 und das Drehmoment 7 sind als zusätzliche Leitungen 8, 9 vorzugsweise Glasleiter durch das Handstück geführt, über die die entstehenden Lasersignale auf Hallsensoren 10, 11 im Schwenkteil 19 des Gehäuses 12 geleitet werden. Dort werden sie in elektrische Signale umgewandelt, welche über die im Versorgungsgerät 28 angeordnete Elektronik den im Gehäuse 12 angeordneten Motor entsprechend steuern.

In den Informationsspeicherringen 6, 7 können Drehzahlen zwischen 0 bis ca. 300.000 bis 400.000 Umdrehungen enthalten sein. Dasselbe gilt für das Drehmoment. Mit diesen Doppelinformationen bzw. mit dem Systeminformationsaufbau von Drehzahl und Drehmoment können viele Bohrerproduzenten, die weltweit vorhanden sind und in Wettbewerb stehen, unabhängig von den vielen Geräte- und Motorherstellern, die Bohrer in dem jetzt vorhandenen weltweiten Vertriebssystem ohne Rücksprache und Abstimmung mit den jeweiligen Geräteherstellern verkaufen.

Da der Zahnarzt bei Verwendung der neuen Doppelinformation weder die Drehzahl noch das Drehmoment in Abhängigkeit vom verwendeten Bohrer selbst einstellen muß, reduziert sich sein Eingriff auf das reine Ein- und Ausschalten. Dieses kann vorteilhafterweise berührungslos (voice dialling) erfolgen, sodaß beim Versorgungsgerät keine Sterilisationsprobleme auftreten.

Für die drehfeste Verbindung zwischen dem Handstück 15 und dem Gehäuse 12 ist im Gehäuse 12 eine Ausnehmung 38 vorgesehen, in die ein Vorsprung 37 des Handstückes 15 eingreift. Die Ausnehmung 38 ist dabei in der Umfangsrichtung größer als der Vorsprung 37, sodaß die für die Schaltung des Motors 31 erforderliche geringfügige Verdrehung erhalten bleibt. Die Ausnehmung 38 erlaubt auch das Anstecken von herkömmlichen Handstücken ohne Leitungsträger.

## Patentansprüche

1. Zahnärztliches Arbeitsgerät, mit einem ein angetriebenes Werkzeug aufnehmenden Handstück, welches durch einen Leitungen enthaltenden Schlauch mit einem Versorgungsgerät verbunden ist, **dadurch gekennzeichnet, daß** der Schlauch (14) drehbar mit dem Versorgungsgerät (28) verbunden ist.

2. Zahnärztliches Arbeitsgerät nach Anspruch 1, **dadurch gekennzeichnet, daß** im Versorgungsgerät (28) ein Antrieb (31) zur Drehung des Schlauches (14) vorgesehen ist.

3. Zahnärztliches Arbeitsgerät nach Anspruch 2, **dadurch gekennzeichnet, daß** zur Steuerung des Antriebs (31) zwei Schalter (20, 21) vorgesehen sind, welche durch einen Schwenkteil (19) betätigbar sind, der in einem mit dem Schlauch (14) drehfest verbundenen Gehäuse (12) gelagert ist.

4. Zahnärztliches Arbeitsgerät nach Anspruch 3, **dadurch gekennzeichnet, daß** der Schwenkteil (19) mit dem Handstück (15) lösbar, aber verdrehfest verbunden ist.

5. Zahnärztliches Arbeitsgerät nach Anspruch 4, **dadurch gekennzeichnet, daß** zwischen dem Handstück (15) und dem einen Motor aufnehmenden Gehäuse (12) eine das Gehäuse (12) und den Schlauch (14) umgebende Hülle (25) festklemmbar ist.

6. Zahnärztliches Arbeitsgerät nach einem der Ansprüche 1 bis 5, **dadurch gekennzeichnet, daß** das Handstück (15) mit Längsrillen (26) versehen ist.

7. Zahnärztliches Arbeitsgerät nach einem der Ansprüche 1 bis 6, **dadurch gekennzeichnet, daß** im Handstück (15) Leitungen (8, 9) zum Ablesen von am Werkzeug (5) angebrachten Informationen geführt sind.

8. Zahnärztliches Arbeitsgerät nach Anspruch 7, **dadurch gekennzeichnet, daß** am Werkzeug (5) Informationsspeicherringe (6, 7) vorgesehen sind, wobei im Ring (6) Informationen zur Drehzahl und im Ring (7) Informationen zum Drehmoment gespeichert sind.

9. Zahnärztliches Arbeitsgerät nach einem der Ansprüche 1 bis 8, **dadurch gekennzeichnet, daß** das Handstück (15) einen abnehmbaren Leitungsträger (13) umfaßt.

10. Zahnärztliches Arbeitsgerät nach Anspruch 9, **dadurch gekennzeichnet, daß** der Leitungsträger (13) Leitungen (1, 3, 3', 4, 4', 22 und 23) enthält, die für die Übertragung von Bilddaten, von Licht und von Werkzeuginformationen vorgesehen sind.
